# EUROPÄISCHE PATENTSCHRIFT

(11) **EP 3 409 783 B1**
(45) Veröffentlichungstag und Bekanntmachung des Hinweises auf die Patenterteilung: **16.12.2020**
(21) Anmeldenummer: 17173895.8
(22) Anmeldetag: 01.06.2017
(51) Int. Cl.: C12Q 1/04, C12N 1/20

(54) **ERFINDUNG BETREFFEND DETEKTION UND QUANTIFIZIERUNG VON COLISTIN-RESISTENZ BEI GRAM-NEGATIVEN BAKTERIEN**
INVENTION RELATING TO DETECTION AND QUANTIFICATION OF COLISTIN RESISTANCE IN GRAM-POSITIVE BACTERIA
INVENTION CONCERNANT LA DÉTECTION ET LA QUANTIFICATION DE LA RÉSISTANCE À LA COLISTINE CHEZ DES BACTÉRIES GRAM NÉGATIVES

(43) Veröffentlichungstag der Anmeldung: 05.12.2018
(73) Patentinhaber: Justus-Liebig-Universität Gießen, 35390 Gießen (DE)
(72) Erfinder: Prof. Chakraborty, Trinad, 35394 Giessen (DE); Falgenhauer, Linda, 35398 Giessen (DE); Gwozdzinski, Konrad, 35398 Giessen (DE); Imirzalioglu, Can, 35641 Schöffengrund-Laufdorf (DE)
(74) Vertreter: Stumpf, Peter

(56) Entgegenhaltungen:
- WO-A1-2014/134071
- US-A1- 2010 086 367
- KONSTANTINA DAFOPOULOU ET AL: "Comparative Evaluation of Colistin Susceptibility Testing Methods among Carbapenem-Nonsusceptible Klebsiella pneumoniae and Acinetobacter baumannii Clinical Isolates", ANTIMICROBIAL AGENTS AND CHEMOTHERAPY, Bd. 59, Nr. 8, 26. Mai 2015 (2015-05-26), Seiten 4625-4630, XP055416811, ISSN: 0066-4804, DOI: 10.1128/AAC.00868-15

### SEQUENCE LISTING

<110> Justus-Liebig-Universität
<120> Erfindung betreffend Detektion und Quantifizierung von
   Colistin-Resistenz bei Gram-negativen Bakterien
<130> EP
<160> 6
<170> BiSSAP 1.3.5
<210> 1
   <211> 32
   <212> DNA
   <213> Artificial Sequence
<223> "mcr-1_forward primer"
<220>
   <223> mcr-1_forward primer
<400> 1
   gcgcgcgtcg acatgagcag catactctgt gt 32
<210> 2
   <211> 31
   <212> DNA
   <213> Artificial Sequence
<223> "mcr-1_reverse primer"
<220>
   <223> mcr-1_reverse primer
<400> 2
   gcgcgcgaat tctcagcgga tgaagcggtg c 31
<210> 3
   <211> 34
   <212> DNA
   <213> Artificial Sequence
<223> "mcr-1_phosphatase primer"
<220>
   <223> mcr-1_phosphatase primer
<400> 3
   gcgcgcgtcg acatgatgca gcatactctg tgtg 34
<210> 4
   <211> 37
   <212> DNA
   <213> Artificial Sequence
<223> "mcr-1_phosphatase primer"
<220>
   <223> mcr-1_phosphatase primer
<400> 4
   gcgcgcgaat tcgatacgac cgtttttact tttaaag 37
<210> 5
   <211> 33
   <212> DNA
   <213> Artificial Sequence
<223> "F_phos_ATGG_pSU2719 primer"
<220>
   <223> F_phos_ATGG_pSU2719 primer
<400> 5
   gcgcgcgtcg acaagggcgg tggggtgggg ttt 33
<210> 6
   <211> 38
   <212> DNA
   <213> Artificial Sequence
<223> "R_phos_kpn1_pSU2719 primer"
<220>
   <223> R_phos_kpn1_pSU2719 primer
<400> 6
   gcgcgcggta ccgatacgac cgtttttact tttaaagg 38

## Beschreibung

Die vorliegende Erfindung betrifft das Gebiet der Mikrobiologie und den zuverlässigen Nachweis von Gram-negativen Bakterien, die eine Resistenz gegenüber dem Antibiotikum Colistin aufweisen.

Colistin (auch Polymyxin E genannt) ist ein Polypeptid-Antibiotikum aus der Gruppe der Polymyxine. Es ist seit 1959 zur Behandlung bakterieller Infektionskrankheiten bekannt. Der Einsatz von Colistin ist auf Gram-negative Bakterien beschränkt, da für die bakterizide, also Bakterien-abtötende Wirkung von Colistin das Vorhandensein von Lipopolysaccharid (LPS) Voraussetzung ist, welches nur in Gram-negativen Bakterien zu finden ist. Colistin wirkt über eine Störung der Permeabilität der Zellmembran von Gram-negativen Bakterien.

Gram-negative Bakterien sind Bakterien, die nicht auf die dem Fachmann bekannte Gramfärbung reagieren bzw. es sind Bakterien, bei denen die Gramfärbung wieder mit Alkohol ausgewaschen werden kann, da diese Bakterien nur eine dünne Mureinhülle ohne Teichonsäuren aufweisen. Beispiele für Gram-negative Bakterien sind alle Arten der Abteilung Proteobacteria, Enterobakterien, wie *E. coli, Salmonella, Shigella, Klebsiella, Proteus, Enterobacter,* sowie die Gattungen *Pseudomonas, Legionella, Neisseria, Rickettsia,* aber auch *Streptobacillus moniliformis, Meningococcus, Chlamydia, Spirochäta, Bacteroidetes, Bartonella, Brucella* und *Coxiella* wie in Standardwerken der Mikrobiologie dargestellt ist (z.B. das Standardwerk Bergey's Manual of Systematics of Archaea and Bacteria, 2015).

Colistin wird in der Humanmedizin als absolutes Reserveantibiotikum angesehen. Es wird insbesondere bei Patienten, die Carbapenem-resistente Enterobakterien (sog. "CRE", z.B. *Escherichia coli, Klebsiella pneumoniae*) aufweisen, verwendet, weil diese CRE hoch-resistent sind. CRE kommen weltweit mit steigender Prävalenz in Europa vor.

Sie werden besonders häufig in Griechenland und Italien isoliert, die als Hochprävalenzländer gelten. Patienten mit CRE können bei Vorliegen einer bakteriellen Infektion nur noch mit sehr wenigen Antibiotika wirksam behandelt werden, z.B. mit Colistin. Die durch die Nephrotoxizität bedingten Nebenwirkungen von Colistin werden dann billigend in Kauf genommen. Um die Nebenwirkungen abzuschwächen, wird dem Patienten Colistin als Prodrug z.B. Colistimethat-Natrium (CMS) verabreicht, welches im Körper durch Hydrolyse in die pharmakologisch aktive Colistin-Base umgewandelt wird. Ein Prodrug ist allgemein ein inaktiver oder wenig aktiver pharmakologischer Stoff, der erst durch Verstoffwechslung im Organismus z.B. durch Hydrolyse in einen aktiven Wirkstoff überführt wird. Die Darreichungsform von Colistimethat-Natrium (CMS) ist entweder inhalativ oder parenteral. Oral wird Colistin nur dann verabreicht, wenn eine Darmbehandlung erforderlich ist.

In der Veterinärmedizin wird Colistin regelmäßig zur Infektionstherapie angewendet. Außerhalb von Europa z.B. in Indien wird Colistin hingegen auch als Futterzusatz in der Tiermast verwendet. Es gibt Bestrebungen auf europäischer Ebene, aufgrund von steigenden Resistenzraten z.B. in der Tiermast, die Verwendung von Colistin zur Infektionstherapie in der Veterinärmedizin möglichst einzudämmen.

Gram-negative Bakterien können jedoch eine Resistenz gegen Colistin ausbilden. Bei Patienten mit Gram-negativen Bakterien, die eine Resistenz gegen Colistin ausgebildet haben, ist eine Antibiotikatherapie mit Colistin nicht mehr wirksam. Wesentliche Mechanismen der Colistinresistenz beinhalten z.B. die Veränderung des Lipopolysaccharids (LPS), Porine, Membranpumpen und Kapseln.

Es wird zwischen zwei unterschiedlichen Arten der Resistenz unterschieden: die intrinsische und die adaptive Resistenz. Unter einer intrinsischen Resistenz versteht man stabil vererbte genetische Eigenschaften, die auch ohne die Anwesenheit eines Antibiotikums vorhanden sind. So kodieren z.B. alle *Serratia marcescens* das Operon *arnBCADTEF*, welches den Lipid A-Anteil des LPS modifiziert und somit diesen Bakterien eine Colistinresistenz verleiht. Unter einer adaptiven Resistenz versteht man eine Colistinresistenz, bei der bestimmte Gene nur in Anwesenheit des Antibiotikums exprimiert werden.

Dieser Mechanismus beinhaltet z.B. die Mutation negativer Regulatoren der LPS-Modifizierung (z.B. *mgrB*), so dass die LPS-Modifizierung dauerhaft stattfindet und somit eine Colistinresistenz besteht. Diese Resistenzmechanismen sind auf dem Chromosom der Bakterien lokalisiert und können somit nur von Mutter- auf Tochterzelle (also vertikal) vererbt werden. Der intrinsischen und adaptiven Resistenz steht erst seit kurzer Zeit eine transferierbare Colistinresistenz gegenüber, die auf sogenannten Plasmiden lokalisiert ist und über horizontalen Gentransfer (Konjugation) übertragen wird. Die hierfür verantwortlichen Gene sind *mcr-1* und *mcr-2* und Varianten dieser beiden Grundtypen. Die Abkürzung *mcr-1* steht für mobile colistin resistance Gen 1, welches für das Enzym MCR-1 kodiert, *mcr-2* steht für mobile colistin resistance Gen 2, welches für das Enzym MCR-2 kodiert. Bioinformatorische Analysen zeigen, dass diese Gene immer mit einer Phosphatase assoziiert sind. Ob diese Phosphatase einen Einfluss auf die Colistinresistenz hat, ist allerdings noch nicht bekannt.

Plasmid-Iokalisierte Colistinresistenzgene können im Gegensatz zu den chromosomal lokalisierten Resistenzen sehr schnell (innerhalb von Stunden) und insbesondere auch Spezies-übergreifend (z.B. von *Escherichia coli* auf *Klebsiella pneumoniae*) übertragen werden. Beide Resistenzgene *mcr-1* und *mcr-2* sind Phosphoethanolamin-Transferasen und modifizieren den Lipid A-Anteil des LPS. Sie wurden bereits in Enterobakterien unterschiedlichen Ursprungs (Mensch, Tier, Lebensmittel, Umwelt) und auch in hoch-resistenten Keimen (CRE) gefunden. Um eine Therapie mit Colistin durchführen zu können, wird zunächst die Resistenz der Bakterien, die bekämpft werden sollen, gegenüber Colistin mit standardisierten Verfahren ermittelt. Hierzu werden die Bakterien in Gegenwart steigender Konzentrationen des Antibiotikums Colistin kultiviert bzw. in geeignetem Medium angezüchtet. Dann wird die sogenannte Minimale Hemm-Konzentration (MHK) ermittelt, d.h. die minimale Konzentration des Antibiotikums, bei der die Bakterien nicht mehr wachsen. Diese MHK wird nun mit festgelegten Standardwerten (sogenannte Clinical breakpoints) verglichen, um festzustellen, ob der ermittelte MHK-Wert als S - sensibel oder R - resistent eingestuft werden soll.

Die Ergebnisse der Resistenzbestimmung dienen dem Mikrobiologen und dem behandelnden Arzt zur Auswahl einer gezielten antibiotischen Therapie und zeigen im Falle eines Ergebnisses von S - sensibel an, dass ein Patient mit Colistin behandelt werden kann. Im Falle eines Ergebnisses von R - resistent sollte ein Patient nicht mit Colistin behandelt werden.

Es gibt prinzipiell viele Methoden, eine Antibiotika-Resistenzbestimmung durchzuführen. Aufgrund der Tatsache, dass es sich bei Colistin um ein Polypeptid handelt, ist die Auswahl der Methoden zur Resistenzbestimmung, laut CLSI-EUCAST joint Polymyxin Breakpoints Working Group allerdings sehr begrenzt. EUCAST ist die Europäischen Kommission zur Testung von antimikrobieller Anfälligkeit, die mit dem CLSI, dem Clinical and Laboratory Standards Institute, die Standards für die Sensibilitätstestung gegenüber Antibiotika festlegt. Diese Standards berücksichtigen klinische und pharmakokinetische Aspekte bei der antimikrobiellen Therapie.

Allgemein anerkannt sind die Agardiffusionsmethoden, d.h. Methoden, bei denen Antibiotika-Plättchen bzw. -Gradientenstreifen (z.B. Epsilometertest) zur Ermittlung auf eine mit dem Bakterienstamm bestrichene Agarplatte aufgelegt werden. Die Struktur des Colistin verfälscht jedoch die Messergebnisse bei Agardiffusionsmethoden erheblich, da die Diffusion von Colistin aufgrund der Molekülgröße in Agarplatten stark eingeschränkt ist. Dilutionsmethoden (oder auch automatisierte Dilutionsmethoden) enthalten Kunststoffe, die Colistin binden und somit ebenfalls das Ergebnis verfälschen. Die einzige von der CLSI-EUCAST joint Polymyxin Breakpoints Working Group empfohlene Methode ist eine Mikrodilutionsmethode, welche das Wachstum von Bakterien in einer Mikrotiterplatte unter verschiedenen Konzentrationen von Colistin analysiert. Laut der CLSI-EUCAST joint Polymyxin Breakpoints Working Group muss ein besonderes Wachstumsmedium, welches eine erhöhte Konzentration an Calcium- (25 mg/L) und Magnesiumionen (12,5 mg/L) enthält (sogenanntes Cation-adjusted Mueller-Hinton-Medium) und besondere Mikrotiterplatten (Polystyrol, low peptide binding capacity) verwendet werden. Mit dieser Methode lassen sich zwar die Resistenzraten der Bakterien sehr gut ermitteln, sie ist aber sehr zeitaufwendig, kaum automatisierbar und daher zurzeit für Mittel- bis Hochdurchsatzanwendungen, wie es z.B. in der mikrobiellen Diagnostik benötigt wird, nicht geeignet.

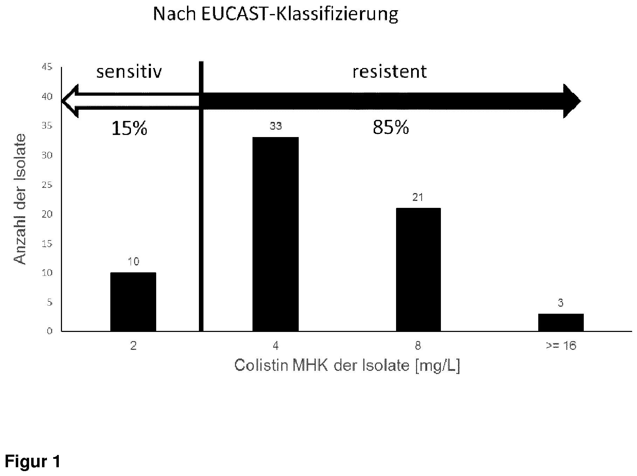

Eine genaue, schnelle und einfache Resistenzbestimmung ist besonders im Falle der durch *mcr-1* bzw. *mcr-2* vermittelten Colistinresistenz extrem wichtig, denn die MHK-Werte dieses Resistenzmechanismus sind bei weitem nicht so hoch wie diejenigen, die über intrinsischen bzw. adaptiven Mechanismen. So kommt es dazu, dass mit Agardiffusionsmethoden MHK-Werte ermittelt werden, die laut der durch EUCAST definierten klinischen Breakpoints in die Kategorie S, also sensibel eingestuft werden (MHK-Werte ≤ 2mg/L, Stand 10.03.2017, http://www.eucast.org/fileadmin/src/media/PDFs/EUCAST_files/Breakpoint_tables /v_7.1_Breakpoint_Tables.pdf). Eine Überprüfung zeigt, dass dies bei bis zu 15% der Isolate der Fall ist, so dass eine hohe falsch-negative Rate vorliegt. Figur 1 zeigt beispielhaft die in eigenen Experimenten ermittelten MHK-Werte von 67 *mcr-1*-kodierenden Enterobakterien-Isolaten.

Falsch-negative Ergebnisse sind aber insbesondere bei diesen mobilen Resistenzen sehr nachteilig, da somit durch eine potentielle Nicht-Erkennung der Resistenz die Übertragung der resistenten Erreger oder Resistenz-vermittelnden Elemente sehr schnell erfolgen kann. Nachteilig an falsch-negativen Ergebnissen ist zusätzlich, dass der Patient mit Colistin behandelt wird, obwohl es nicht wirksam ist.

### Aufgabe

Aufgabe der vorliegenden Erfindung ist es, ein einfaches, leicht durchführbares, kostengünstiges und vor allem zuverlässiges Verfahren zur Detektion von Colistin-resistenten Gram-negativen Bakterien bereitzustellen. Es soll die Möglichkeit geben, spezifisch, zuverlässig und leicht durchführbar zwischen sensiblen und resistenten Gram-negativen Bakterien zu unterscheiden. Es sollen sowohl Gram-negative Bakterien mit Plasmid-Iokalisierter Colistinresistenz als auch Gram-negative Bakterien mit chromosomal lokalisierter Resistenz detektiert werden können. Das Verfahren soll im Hochdurchsatz in der mikrobiologischen Diagnostik verwendet werden können.

Eine genaue, schnelle und einfache Resistenzbestimmung ist besonders im Falle der durch *mcr-1* bzw. *mcr-2* vermittelten Colistinresistenz extrem wichtig, denn die MHK-Werte dieses Resistenzmechanismus sind bei weitem nicht so hoch wie diejenigen, die über intrinsischen bzw. adaptiven Mechanismen. So kommt es dazu, dass mit Agardiffusionsmethoden MHK-Werte ermittelt werden, die laut der durch EUCAST definierten klinischen Breakpoints in die Kategorie S, also sensibel eingestuft werden (MHK-Werte ≤ 2mg/L, Stand 10.03.2017, http://www.eucast.org/fileadmin/src/media/PDFs/EUCAST_files/Breakpoint-tables /v_7.1_Breakpoint_Tables.pdf). Eine Überprüfung zeigt, dass dies bei bis zu 15% der Isolate der Fall ist, so dass eine hohe falsch-negative Rate vorliegt. Figur 1 zeigt beispielhaft die in eigenen Experimenten ermittelten MHK-Werte von 67 *mcr-1*-kodierenden Enterobakterien-Isolaten.

Falsch-negative Ergebnisse sind aber insbesondere bei diesen mobilen Resistenzen sehr nachteilig, da somit durch eine potentielle Nicht-Erkennung der Resistenz die Übertragung der resistenten Erreger oder Resistenz-vermittelnden Elemente sehr schnell erfolgen kann. Nachteilig an falsch-negativen Ergebnissen ist zusätzlich, dass der Patient mit Colistin behandelt wird, obwohl es nicht wirksam ist.

Im Stand der Technik beschreibt US2010/0086367A1 ein Verfahren zur Verbesserung der Widerstandsfähigkeit eines porösen oder durchlässigen Materials, wobei *Sporocarcina pasteurii* mit einem Medium in Kontakt gebracht wird, welches 5,7 mM oder 10 mM Calcium sowie 10 µM NiCl₂ aufweist.

WO2014/134071A1 beschreibt die Herstellung von rekombinanten Proteinen in Zellen. Zur Stabilisierung der Struktur des rekombinanten Proteins wird dem Medium Mannose- oder Calcium zugefügt. Es wird eine Calciumkonzentration in einem Bereich von etwa 1,5 mM bis etwa 9,5 mM eingesetzt.

### Lösung der Aufgabe

Diese Aufgabe wird erfindungsgemäß gelöst durch ein Verfahren gemäß Anspruch 1.

Dieses Verfahren zur Detektion von Colistin-resistenten Gram-negativen Bakterien in einer biologischen Probe umfassend folgende Schritte:
i) Bereitstellen eines sterilen für Gram-negative Bakterien geeigneten Nährmediums umfassend 2,5 - 20 mM Calciumionen
ii) Zugabe von Colistinsulfat in einer Konzentration von 0,125 mg/L - 512 mg/L zum Medium aus Schritt i)
iii) Zugabe einer Reinkultur eines Gram-negativen Bakteriums gewonnen aus einer biologischen Probe zum Medium nach Schritt ii)
iv) Inkubieren des Mediums mit der Reinkultur eines Gram-negativen Bakteriums nach Schritt iii)
v) Detektion von Colistin-resistenten Gram-negativen Bakterien bei Wachstum in Medium aus i) und einer Colistinsulfat-Konzentration ≥ des klinischen breakpoint.

Erstmals steht damit ein einfaches, leicht durchführbares, kostengünstiges und vor allem zuverlässiges Verfahren zur Detektion von Colistin-resistenten Gram-negativen Bakterien in einer biologischen Probe zur Verfügung, mit dem es möglich ist, zwischen sensiblen und resistenten Gram-negativen Bakterien zu unterscheiden. Das Verfahren ist im Hochdurchsatz in der klinischen Diagnostik einsetzbar. Darüber hinaus verbessert das Verfahren Agardiffusions-basierte Methoden zur Colistinresistenztestung, die aufgrund der Eigenschaften von Colistin durch die CLSI-EUCAST joint Polymyxin Breakpoints Working Group als nicht verwendbar klassifiziert worden sind.

Die Erfindung hat den Vorteil, dass sie in die bereits bestehenden Verfahren der Europäischen Kommission zur Testung von antimikrobieller Anfälligkeit (EUCAST) eingefügt werden kann. Die erstellten Standards werden damit erfüllt. Agardiffusions-basierte Testverfahren zur Bestimmung der Colistinresistenz, die aufgrund ihrer schlechten Leistungsfähigkeit als ungeeignet eingestuft worden sind, können damit wiederaufgenommen werden.

Die Erfindung hat weiterhin den Vorteil, dass Bakterien mit Plasmid-Iokalisierter Colistinresistenz und auch Bakterien mit chromosomal lokalisierter Resistenz detektiert werden. Somit werden falsch negative Ergebnisse vermieden. Die Colistin-resistenz konnte bislang in Bakterien mit Plasmid-Iokalisierter Colistinresistenz nur mit erhöhtem Aufwand in speziellen Laboren nachgewiesen werden.

Ein wesentlicher Aspekt der Erfindung besteht in der Bereitstellung eines Nährmediums bzw. Kulturmediums, das erfindungsgemäß Calciumionen aufweist.

Das erfindungsgemäße Nährmedium wird daher im Folgenden als "Calcium-enhanced Colistin-resistance detection Medium" (CECRDM) bezeichnet. Es kann als festes oder flüssiges Nährmedium ausgebildet sein. Allgemein dienen Nährmedien zum Wachstum oder zur Kultivierung von Mikroorganismen. Flüssige Nährmedien werden dabei auch als Medium, Bouillon, Nährbouillon oder Nährlösung bezeichnet. Feste Nährmedien sind durch Zusatz eines Geliermittels geliert bzw. gelförmig und werden auch als Nährboden bezeichnet. Feste Nährmedien werden bevorzugt zu Analysezwecken und zur Quantifizierung von Bakterien verwendet, da sich die Bakterien nicht frei im Nährmedium verteilen können und bei ihrer Vermehrung auf dem Nährmedium eine sichtbare, gut zählbare Kolonie bilden. Im Idealfall entspricht die Anzahl der Kolonien der ursprünglichen Anzahl von Mikroorganismen. Da jedoch eine Kolonie auch von mehreren dicht beieinanderliegenden Individuen gebildet werden kann, entspricht die Anzahl der Kolonien nicht wirklich der tatsächlichen Anzahl der Bakterien, sondern nur der Anzahl der Individuen und der in Gruppen im oder auf dem Nährboden verteilten Bakterien. Diese Individuen und Gruppen bezeichnet man als KBE (**K**olonie**b**ildende **E**inheit oder engl. cfu - **c**olony **f**orming **u**nits).

Die prinzipielle Herstellung von Nährmedien ist im Stand der Technik beschrieben und dem Fachmann bekannt. Ein Nährmedium besteht meist aus einem Hauptanteil Wasser sowie Nähr- und Zusatzstoffen. Diese umfassen mindestens eine, für den jeweiligen Organismus verwertbare Energiequelle, wie z.B. organische oder schwefelartige Verbindungen sowie Nährstoffe in Form von organischen oder anorganischen C-, N-, S- und P-Quellen. Nährstoffe sind auch Kohlenhydrate, Proteinhydrolysate und ggf. Fettsäuren sowie anorganische Ionen wie NH₄⁺, K⁺, Na⁺, SO₄⁻, PO₄³⁻, und Spurenelemente.

Es können weiterhin Zusatzstoffe, wie z.B. Farbstoffe, Geliermittel (z.B. Agar-Agar, Gelatine), Hemmstoffe (z.B. Antibiotika), pH-Indikatoren, Puffersubstanzen, um den pH-Wert zu stabilisieren sowie Wachstumsfaktoren, Hormone oder Vitamine zugegeben werden. Flüssigmedien ähneln in der Zusammensetzung den festen Nährmedien, nur ist bei ihnen kein Agar-Agar oder ein anderes Geliermittel enthalten. Feste Nährmedien enthalten mindestens 1 % Agar-Agar, je nach Rezeptur variiert die Agar-Agar Konzentration zwischen 10 - 20 g/L Nährmedium. Für einen Weichagar ist eine Agar-Agar Konzentration 1 - 4 g/L üblich.

Bekannt ist das Cation-adjusted Mueller-Hinton-Medium (CAB), welches zurzeit für die Bestimmung einer Colistinresistenz eingesetzt wird. Es enthält Cationen mit einer Zusammensetzung von 20 - 25 mg/L Calciumionen (entsprechend 0.5 -0.6 mM) und 10 - 12.5 mg/L Magnesiumionen.

Das erfindungsgemäße Nährmedium "Calcium-enhanced Colistin-resistance detection Medium" (CECRDM) weist gegenüber den bekannten Nährmedien eine Besonderheit auf, nämlich, dass die Calciumkonzentration deutlich über der Konzentration des Cation-adjusted Mueller-Hinton-Mediums (CAB) liegt. Basierend auf Vergleichsexperimenten kann gezeigt werden, dass die erfindungsgemäße Aufgabe nicht mit Cation adjusted Mueller-Hinton-Medium (CAB) gelöst werden kann. So zeigt Figur 1 die Überprüfung von 67 mcr-1-kodierenden Enterobakterien-Isolaten, deren MHK-Werte mittels Agardiffusionsmethoden (Epsilometertest) in Cation-adjusted Mueller-Hinton-Medium (CAB) ermittelt wurden. Gemäß der durch EUCAST definierten klinischen Breakpoints (Stand 10.03.2017) wurden 15% der mcr-1-kodierenden Enterobakterien in die Kategorie S, also sensibel eingestuft (MHK-Werte ≤ 2mg/L). Es liegt also eine hohe falsch-negative Rate vor. Unter Verwendung des erfindungsgemäßen Nährmediums "Calcium-enhanced Colistin-resistance detection Medium" (CECRDM) wiesen alle 67 mcr-1-kodierenden Enterobakterien-Isolate MHK-Werte ≥4 mg/L auf.

Das erfindungsgemäße Medium "Calcium-enhanced Colistin-resistance detection Medium" (CECRDM) kann als festes oder flüssiges Nährmedium verwendet werden. Es weist erfindungsgemäß Calciumionen im Bereich von 2,5 - 20 mM auf, als besonders bevorzugt konnte 5 mM Calcium ermittelt werden.

Die Herstellung von CECRDM erfolgt z.B. über die Zugabe von Calciumionen zu einem festen oder flüssigen Standard-Medium, das für Gram-negative Bakterien geeignet ist, wie z.B. LB-Medium, Mueller-Hinton-Medium oder chromogene Medien. Dabei werden Calciumionen in einer Konzentration von 200-800 mg/L (entsprechend 2,5-20 mM) zugegeben. In einer bevorzugten Ausführungsform wird Calciumchlorid-Dihydrat zugefügt, um eine Calciumionen-Konzentration von 200-800 mg/L (entsprechend 2,5-20 mM Calciumionen) zu erhalten. In einer weiteren bevorzugten Ausführungsform wird die Calciumionen-Konzentration von 200 mg/L (entspricht 5 mM Calciumionen) eingestellt.

Mit diesem CECRDM-Medium ist das Nachweisverfahren für Colistin-resistente Gram-negative Bakterien in einer biologischen Probe möglich. Insbesondere ist auch der sichere Nachweis von Gram-negativen Bakterien, die eine Plasmidlokalisierte Colistinresistenz tragen, möglich.

Als biologische Probe wird im Folgenden jegliches Material eines Menschen oder Tieres verstanden, wie die Körperflüssigkeiten Vollblut, Serum oder Plasma, Seminalplasma, Knochenmark, Knochenmarkspunktate, Spinalflüssigkeit, peritoneale Flüssigkeit, Speichel, Tränenflüssigkeit, zellhaltige Materiale (z.B. Stammzellpräparate) oder Urin.

Weiterhin ist eine biologische Probe auch Material einer Stuhlprobe, Biopsiematerial oder Gewebe aber auch Abstriche von Körperoberflächen. Eine biologische Probe umfasst auch Boden-, Wasser- und Umweltproben, Gegenstände, Bewuchsproben, die als Wisch-, Pulver-, Fest- oder Flüssigprobe genommen werden.

Die in der biologischen Probe befindlichen Bakterien werden zunächst selektiert, sodass nur die Gram-negativen Bakterien auf Colistinresistenz überprüft werden. Gram-positive Bakterien werden nicht auf Colistinresistenz überprüft, da sie aufgrund ihres Zellaufbaus eine intrinsische Colistinresistenz besitzen.

Überprüft werden alle in den biologischen Proben enthaltenen Gram-negativen Bakterien insbesondere die Abteilung Proteobacteria, Enterobakterien, wie *E. coli, Salmonella, Shigella, Klebsiella, Proteus, Enterobacter*, sowie die Gattungen *Pseudomonas, Legionella, Neisseria, Rickettsia*, aber auch *Streptobacillus moniliformis, Meningococcus, Chlamydia, Spirochäta, Bacteroidetes, Bartonella, Brucella* und *Coxiella.*

### Ausführungsbeispiele

### 1. Bioinformatorische Analyse und Klonierung des mcr-1-Operons

Die Voraussetzung, eine verbesserte Methode zur Detektion der Colistinresistenz zu finden, war die Tatsache, dass etwa 15% aller *mcr-1*-Isolate aus biologischen Proben durch Resistenzbestimmung mittels Agardiffusion fälschlicherweise als Colistin-sensitiv darstellt werden. Es gibt also eine hohe Zahl falscher Messergebnisse, die damit auch zu nicht-adäquaten Therapieempfehlungen führen. Der Vergleich von Literaturdaten mit eigenen Messdaten (Sequenzanalyse über BLAST) zeigte, dass das *mcr-1-*Gen immer mit einer putativen Phosphatase vergesellschaftet ist. Die Vermutung war, dass es sich bei dieser Phosphatase um ein Enzym handelt, welches die durch *mcr-1* vermittelte Colistinresistenz noch erhöhen kann. Dies konnte in der Literatur allerdings nicht bestätigt werden und ist im Stand der Technik nicht bekannt.

Eine genauere Analyse der Erfinder zeigte, dass die Phosphatase ein hypothetisches Calcium-Bindemotiv trägt (Aminosäuresequenz G-G-X-G-X-(D/N)-X-U-X wobei X irgendeine Aminosäure darstellt und U eine hydrophobe Aminosäure), so dass die Vermutung besteht, dass eine Verstärkung der Colistinresistenz nur in Anwesenheit von Calcium möglich sein könnte. Aus diesem Grunde wurde das *mcr-1* Gen alleine, *mcr-1* Gen mit Phosphatase Gen *pap* und das Phosphatase Gen *pap* alleine nach einer dem Fachmann bekannten Vorgehensweise kloniert. Dabei wurde das *mcr-1* Gen alleine bzw. das *mcr-1* Gen mit Phosphatase mittels PCR und Verwendung des *mcr-1*-kodierenden Isolates *E. coli* V163 als template amplifiziert.

V163 ist offenbart in Falgenhauer L, Waezsada S-E, Yao Y, Imirzalioglu C, Käsbohrer A, Roesler U, Michael GB, Schwarz S, Werner G, Kreienbrock L, Chakraborty T. 2016. Colistin resistance gene mcr-1 in extended-spectrum β-lactamase-producing and carbapenemase-producing Gram-negative bacteria in Germany. Lancet Infect Dis 16:282-283.

Als Primer wurden verwendet (die Restriktionsschnittstellen sind unterstrichen):

| **Klonierung** | **Name des Primers** | **Sequenz 5'-3'** | **Seq ID** |
|---|---|---|---|
| *mcr-1* Gen alleine | *mcr-1*_forward | gcgcgcgtcgacatgagcagcatactctgtgt | 1 |
| | *mcr-1*_reverse | gcgcgcgaattctcagcggatgaagcggtgc | 2 |
| *mcr-1* Gen mit Phosphatase Gen pap | *mcr-1*_phosphatase _forward | gcgcgcgtcgacatgatgcagcatactctgtgtg | 3 |
| | *mcr-1*_phosphatase _reverse | gcgcgcgaattcgatacgaccgtttttacttttaaag | 4 |
| Phosphatase Gen pap | F_phos_ATGG_pSU2 719 | gcgcgcgtcgacaagggcggtggggtggggttt | 5 |
| | R_phos_kpn1_pSU27 19 | gcgcgcggtaccgatacgaccgtttttacttt- taaagg | 6 |

Für die Klonierung des *mcr-1* Gens wurden die resultierenden Amplikons mittels der Restriktionsenzyme SalI und EcoRI geschnitten und mit einem SalI/EcoRI geschnittenen pUC19 Plasmid als Vektor ligiert. Das Ligationsprodukt wurde in *E*. *coli* DH10β transformiert. Die Verfahren sind dem Fachmann bekannt.

Figur 2 zeigt die Plasmidkarte für das Konstrukt mit dem *mcr-1* Gen in pUC19. Figur 3 zeigt die Plasmidkarte für das Konstrukt mit dem *mcr-1* Gen und dem Phosphatase-Gen in pUC19 als Vektor.

Für die Klonierung des Phosphatase-Gens *pap* wurde *E. coli* V163 als template benutzt. Die PCR-Fragmente wurden mittels der Restriktionsenzyme SalI und Kpnl geschnitten, in Sall/Kpnl geschnittenes pSU2179 Plasmid als Vektor ligiert und das Ligationsprodukt in *E. coli* DH10β transformiert.

Fig. 4 zeigt die Plasmidkarte für das Konstrukt mit dem Phosphatase-Gen in Vektor pSU2719.

Die Transformanten wurden in "Calcium-enhanced Colistin-resistance detection Medium" (CECRDM) mit verschiedenen Calcium-Konzentrationen von 2,5 - 20 mM angezüchtet, um zu ermitteln, ob das Vorhandensein der Phosphatase bei einer bestimmten Calciumkonzentration die Colistinresistenz erhöhen würde. Die Resistenzbestimmung wurde im Vergleich zu dem Referenzmedium (Cation adjusted Mueller-Hinton-Medium, CAB) durchgeführt. In Tabelle 1 ist das Ergebnis unter Zugabe von 5 mM Calciumionen beispielhaft dargestellt.

Die Zugabe von Calcium in einer Konzentration von 5 mM führte bei Konstrukten mit *mcr-1* immer zu einem Anstieg der MHK (Tabelle 1) um einen Faktor von mindestens 4. Dieser Anstieg war bei *mcr-1* mit Phosphatase ausgeprägter als ohne Phosphatase und war in CAB-Medium nicht zu beobachten.

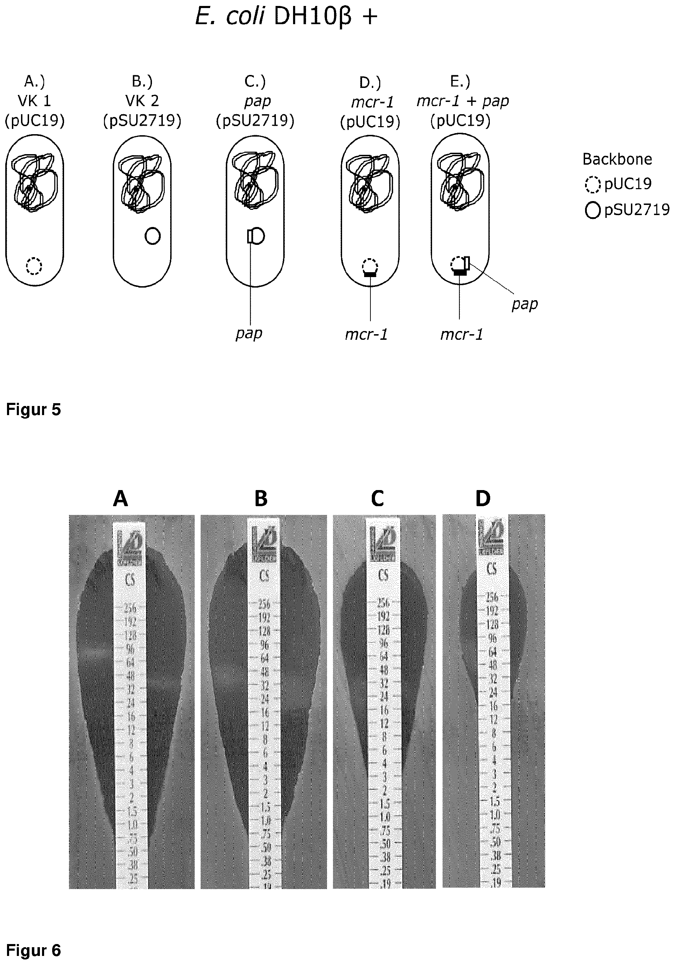

Figur 5 zeigt eine schematische Darstellung der analysierten Konstrukte.
A.) zeigt die Vektorkontrolle VK mit Vektor pUC19.
B.) zeigt die Vektorkontrolle VK mit Vektor pSU2719.
C.) zeigt das Konstrukt mit dem Phosphatase Gen *pap* alleine.
D.) zeigt das Konstrukt mit dem *mcr-1* Gen alleine.
E.) zeigt das Konstrukt mit dem *mcr-1* Gen und dem Phosphatase Gen *pap.*

**Tabelle 1** zeigt die minimalen Hemm-Konzentrationen (MHK) der *mcr-1-*Konstrukte, bestimmt mittels Mikrodilution. Die Benennung A-E erfolgt entsprechend den Konstrukten in Figur 5.

| **Stamm** | **CAB Medium [mg/L]** | **CECRDM Medium [mg/L]** |
|---|---|---|
| ***A.)*** | **0,5** | **0,5** |
| ***B.)*** | **0,5** | **0,5** |
| ***C.)*** | **0,5** | **1** |
| ***D.)*** | **2** | **8** |
| ***E.)*** | **2** | **16** |

Die Ergebnisse der Resistenzbestimmung in CECRDM sind beispielhaft für 5 mM Calcium dargestellt. Sie sind ebenfalls zu beobachten bei 2,5 mM, 3 mM, 4 mM, 5mM, 6 mM, 7 mM, 8 mM, 9 mM, 10 mM, 11mM, 12 mM, 13 mM, 14 mM, 15 mM, 16 mM, 17 mM, 18 mM, 19 mM, 19,5 mM und 20 mM Calcium.

### 2. Herstellen des Calcium-enhanced Colistin-resistance detection Medium, CECRDM

Das erfindungsgemäße Nährmedium "Calcium-enhanced Colistin-resistance detection Medium" (CECRDM) wird hier beispielhaft aus Mueller-Hinton-Medium durch Zugabe von Calcium hergestellt.

Alternativ können auch andere Medien die für Gram-negative Bakterien geeignet sind, verwendet werden, z.B. LB-Medium oder chromogene Medien. Mueller-Hinton-Medium ist kommerziell erhältlich und weist folgende Zusammensetzung auf:
- 1 % Rindfleischaufguss
- 1,75 % hydrolysiertes Casein
- 0,15 % Stärke

Der pH-Wert wird bei 25°C neutral eingestellt. Das Nährmedium wird als Flüssigmedium hergestellt. Es kann jedoch auch als festes Nährmedium hergestellt werden, indem es z.B. mit 1,5% Agar-Agar (w/v) versetzt wird. Das Nährmedium wird sterilisiert z.B. durch Autoklavieren (121°C für 15 Minuten). Nach Abkühlen des sterilen Nährmediums auf 45-50°C werden dem Nährmedium Calciumionen (sterilfiltierte Lösung) beigefügt. Dabei werden Calciumionen in einer Konzentration von 200-800 mg/L (entsprechend 2,5-20 mM) zugegeben.

In einer Ausführungsform wird Calciumchlorid-Dihydrat (CaCl₂ · 2 H₂O) in einer Konzentration von 367.5 - 2940 mg/L zugefügt, um eine Calciumionen-Konzentration von 200-800 mg/L (entsprechend 2,5-20 mM Calciumionen) zu erhalten. In einer bevorzugten Ausführungsform wird die Calciumionen-Konzentration von 200 mg/L (entspricht 5 mM Calciumionen) eingestellt. Alternativ kann Calcium auch als Calciumchlorid-Tetrahydrat (CaCl₂ · 4 H₂O) und/oder als Calciumchlorid-Hexahydrat (CaCl₂ · 6 H₂O) eingesetzt werden. Die eingestellte Calcium-Konzentration übersteigt in jedem Fall deutlich diejenige des Cation adjusted Mueller-Hinton-Medium (CAB), welches zurzeit der Standard für die Bestimmung einer Colistinresistenz ist. Mittels diesem CECRDM-Medium ist das Nachweisverfahren für Colistin-resistente Gram-negative Bakterien insbesondere von Colistin-resistenten Gram-negativen Bakterien in einer biologischen Probe möglich. Insbesondere ist auch der sichere Nachweis von Gram-negativen Bakterien möglich, die eine Plasmid-Iokalisierter Colistinresistenz.

### 3. Resistenzbestimmung

### Herstellung bzw. Bearbeitung des Ausgangsmaterials

Zur Validierung des Verfahrens für die Bestimmung der Colistinresistenz wird als Ausgangsmaterial eine Bakterienreinkultur verwendet und auf dem erfindungsgemäßen "Calcium-enhanced Colistin-resistance detection Medium" (CECRDM) angezüchtet.

Stammt das Ausgangsmaterial aus einer festen oder flüssigen biologischen Probe, wird zunächst eine Bakterienmischkultur aus dieser Probe auf festem Nährmedium (z.B. LB-Medium oder MacConkey-Medium) ausgestrichen. Diese Mischkultur wird so lange vereinzelt, bis mindestens eine Reinkultur vorliegt. Es erfolgt eine Selektion der Gram-negativen Bakterien, denn nur diese werden der Resistenzbestimmung unterzogen. Jede Reinkultur an Gram-negativen Bakterien muss separat auf Colistinresistenz getestet werden. Bei einer Bakterienmischkultur ist eine Aussage über die Resistenz nur grob, aber nicht ausreichend sicher möglich. Eine feste biologische Probe wird zunächst nach üblichen Verfahren verflüssigt, z.B. indem sterile Lösung wie Wasser oder eine geeignete Pufferlösung zugegeben wird.

Die Detektion von Colistin-resistenten Bakterien erfolgt auf festem "Calcium-enhanced Colistin-resistance detection Medium" (CECRDM) unter Zugabe von Colistin entweder mittels Agardiffusionstest z.B. unter Verwendung sogenannter E-Test-Streifen. Alternativ erfolgt die Resistenzbestimmung als Dilutionsverfahren, z.B. als Mikrodilution in einer 96 well-Mikrotiterplatte.

### Detektion Colistin-resistenter Bakterien

Für die Detektion von Colistin-resistenten Bakterien, insbesondere solchen mit plasmid-kodierten Colistinresistenzen, wird CECRDM mit 1,5% Agar-Agar nach Autoklavieren auf ca. 60°C abgekühlt. Anschließend wird Colistinsulfat in einer Konzentration ≥ des klinischen breakpoint (Stand 10.03.2017: 4 mg/L, http://www.eucast.org/fileadmin/src/media/PDFs/EUCAST_files/Breakpoint_tables /v_7.1_Breakpoint_Tables.pdf) zugegeben. Die Platten werden bis zu der Verwendung bei 4°C gelagert. Auf die Platten werden die zu testenden Bakterien als Verdünnungsausstrich ausgestrichen und 16 - 20 h bei 37°C bebrütet.

### Resistenzbestimmung mittels fester Nährmedien

Für den Test der Colistinresistenz auf festem Nährmedium wird CECRDM mit 1,5% Agar-Agar nach Autoklavieren auf ca. 45-50°C abgekühlt. 20 - 25 ml dieses Mediums werden dann in eine sterile Petrischale transferiert, bis zum Verfestigen stehen gelassen und dann bei 4°C bis zur Verwendung gelagert. Die Platten können bis zu drei Monaten gelagert werden.

Für den Test der Colistinresistenz werden so viele Bakterienkolonien von einer z.B. 12 h bebrüteten Reinkultur in 0,9% Natriumchlorid-Lösung resuspendiert, um einen sogenannten McFarland Standard von 0,5 zu erhalten. Diese Suspension wird z.B. mittels Tupfer gleichmäßig auf eine Platte verteilt und trocknen gelassen. Auf diese Platte wird ein Colistin-E-Test-Streifen (z.B. Liofilchem, Range 0.016 - 256 µg/ml, Bestellnummer 92141, 921411 oder 921410) vorsichtig transferiert. Bei diesem Colistin-E-Test handelt es sich um einen sogenannten Epsilometertest (kurz: E-Test). Diese Methode ist dem Fachmann der Mikrobiologie bekannt, um die minimale Hemmkonzentration (MHK) eines Antibiotikums, in diesem Fall Colistin, für ein bekanntes Bakterium zu bestimmen. Insbesondere wird dieser Test verwendet, um das Resistenzverhalten eines Bakteriums zu ermitteln.

Der Test besteht aus einem Kunststoffstreifen, der auf einer Seite mit Colistin versehen ist. Die Konzentration von Colistin steigt exponentiell in Stufen vom unteren zum oberen Ende des Streifens hin an, was auf dem Streifen mit Skalierungen gekennzeichnet ist. Der Streifen wird auf die Bakterienkultur aus der biologischen Probe bzw. einer Referenzkultur gelegt. Während einer Inkubationszeit von 16 - 24 h bei 37°C diffundiert das Colistin in das Medium und hemmt in einer bestimmten Zone das Bakterienwachstum. Anhand dieser Hemmzone und den Skalierungen ist die minimale Hemmkonzentration ablesbar, die für eine effektive Hemmung benötigt wird. Figur 6 zeigt beispielhaft den E-Test zur Bestimmung der Colistinresistenz. Dabei ist sensitives Isolat ohne Calcium 0 mM (A) oder mit 5 mM Calcium (B), resistentes Isolat ohne Calcium 0 mM (C) oder mit 5 mM Calcium (D) zu sehen und die Ergebnisse der Resistenzbestimmung sind in Tabelle 2 dargestellt.

**Tabelle 2 Beispielhafte Darstellung der Bestimmung der MHK-Werte aus Figur 6**

| | **Zugabe von Calcium** | |
|---|---|---|
| | 0 mM | 5 mM |
| Sensitives Isolat | 1 | 1 |
| Resistentes Isolat | 4 | 24 |

Nach der Inkubation wird die Minimale Hemm-Konzentration (MHK) nach den Hersteller- und EUCAST bzw. CLSI-Vorgaben bestimmt. Wie aus Figur 6 ersichtlich, ist die MHK bei einem sensitiven Isolat nicht von der Calcium-Zugabe beeinflusst, wohingegen die Zugabe von Calcium im Falle des resistenten Isolates zu einer Erhöhung der Resistenz, und somit zu einer verbesserten Detektion der Colistinresistenz führt.

### Resistenzbestimmung mittels flüssiger Nährmedien

Zur Resistenzbestimmung werden Mikrotiterplatten verwendet (bevorzugt 96 well Format). Das Verwenden von Polystyrol-Mikrotiterplatten ist zwingend notwendig, da Mikrotiterplatten aus anderen Materialien Colistin binden und daher das Ergebnis verfälschen würden.

Das Ausgangsmaterial für die Resistenzbestimmung mittels flüssiger Nährmedien ist eine Reinkultur eines Gram-negativen Bakteriums, die zuvor aus einer biologischen Probe gewonnen wurde oder die eine Referenzprobe ist. Die Resistenzbestimmung mittels flüssiger Nährmedien wird gemäß EN ISO 20776-1:2006 durchgeführt, wobei anstelle von CAB Medium CECRDM verwendet wird.

**Tabelle 3** zeigt das verwendete Pipettierschema für die Colistinresistenzbestimmung im Mikrotiterplatten-Format. GC stellt die Wachstumskontrolle dar, sie weist Medium mit Bakterien eines Teststammes als Inokulum ohne Antibiotikum auf.

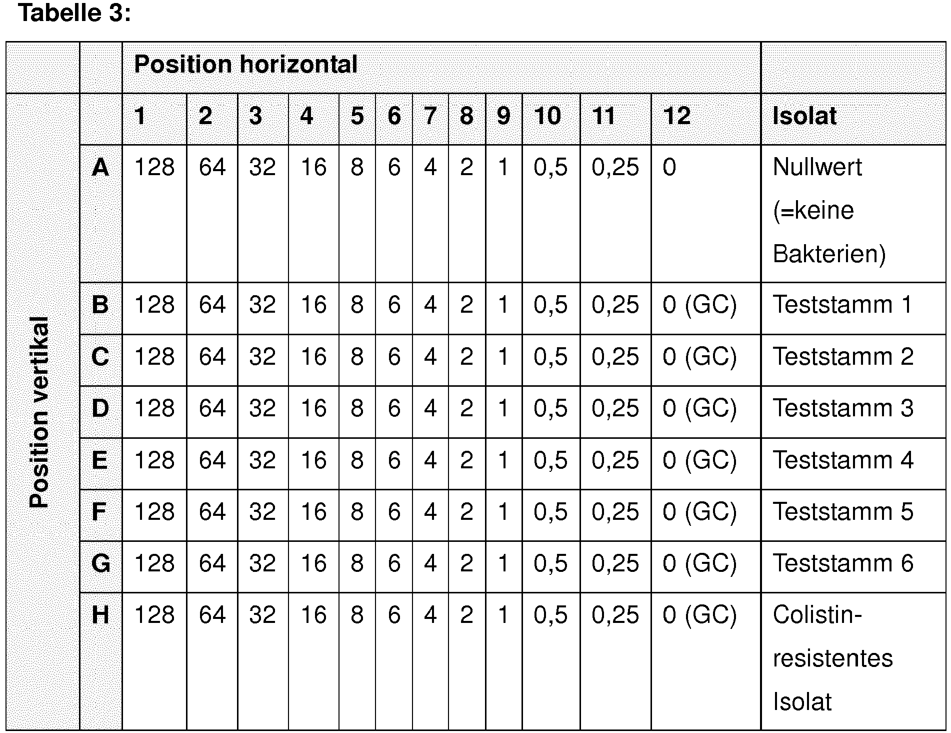

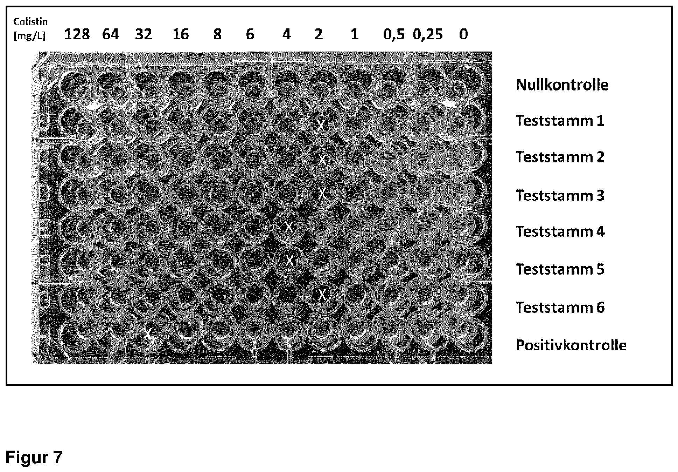

Das Auslesen der Ergebnisse erfolgt über die visuelle Kontrolle des Wachstums. Die MHK wird bestimmt als die Konzentration von Colistin, bei der die Bakterien nicht mehr wachsen. Figur 7 zeigt eine beispielhafte Darstellung der Resistenzbestimmung mittels Mikrodilution im Mikrotiterplattenformat nach der Inkubation. Mit einem weißen X markiert ist die Konzentration, die als MHK bestimmt worden ist. Tabelle 4 zeigt die Auswertung in den Teststämmen 1-6.

**Tabelle 4: Darstellung der aus Figur 7 abgelesenen MHK-Werte**

| **Isolat** | **MHK [mg/L]** |
|---|---|
| Teststamm 1 | 2 |
| Teststamm 2 | 2 |
| Teststamm 3 | 2 |
| Teststamm 4 | 4 |
| Teststamm 5 | 4 |
| Teststamm 6 | 2 |
| Colistin-resistentes Isolat | 32 |

### 4. Test der beschriebenen Methoden an klonierten Colistinresistenzgenen und Wildtypstämmen

Beide Methoden zur Resistenzbestimmung wurden zum einen mit dem in *E. coli* klonierten *mcr-1-*Gen, zum anderen mit 67 Wildtypisolaten aus biologischen Proben von Nutztier, Mensch oder, Lebensmittel, aus einer Stuhlprobe, Biopsiematerial, Boden-, Wasser- oder Umweltprobe mit *mcr-1* bzw. intrinsisch vorhandenen Colistinresistenzen getestet und mit der Referenzmethode (Cation-adjusted Mueller Hinton Medium, CAB) verglichen. Besonders wurde darauf geachtet, ob der einfach durchzuführende Agardiffusionstest (Epsilometertest) unter der erfindungsgemäßen Calciumzugabe vergleichbare Ergebnisse zu der Referenzmethode liefern würde.

Auch im flüssigen Nährmedium sind die MHK-Werte im Vergleich zum Referenzmedium CAB höher und können somit besser ausgelesen werden. Im Vergleich zum Referenzmedium CAB führte die Verwendung von CECRDM zu einer Erhöhung der Colistinresistenz nur im Falle eines Resistenzphänotyps, und somit zu einer verbesserten Detektion der Colistinresistenz. Ausgewählte Beispiele sind in Tabelle 5 aufgeführt. Alle Isolate, die mit der Referenzmethode als sensitiv klassifiziert worden sind, werden auch mittels der neuen Methode als sensitiv eingestuft (MHK ≤ 2 mg/L).

Tabelle 5 zeigt die Ergebnisse der Colistinresistenzbestimmung im Mikrotiterplatten-Format im Vergleich zum E-Test-Streifen-Versuch.

CAB = Cation-adjusted Mueller Hinton-Medium, CECRDM = Calcium-enhanced Colistin-resistance detection Medium, VK= Vektorkontrolle (pUC19 in *E. coli* DH10β) *Colistin sensitiv, ** intrinsische Colistinresistenz, *pap* Phosphatase aus dem *mcr-1*-Operon

**Tabelle 5: Bestimmung der MHK gegenüber Colistin im Mikrotiterplatten-Format bzw. E-Test-Streifen-Versuch.**

| | **MHK über Mikrodilution [mg/L]** | | **MHK über E-Test [mg/L]** | |
|---|---|---|---|---|
| **Stamm** | CAB | CECRDM | CAB | CECRDM |
| ***E. coli DH10β VK*** | 0,5 | 0,5 | 1 | 1 |
| ***E. coli DH10β mcr-1*** | 2 | 8 | 2 | 3 |
| ***E. coli DH10β mcr-1*+*pap*** | 2 | 16 | 3 | 6 |
| ***E. coli* S-105-1** | 8 | 32 | 6 | 32 |
| ***E. coli* RL138** | 4 | 16 | 4 | 16 |
| ***E. coli* S-462-1** | 8 | 32 | 6 | 32 |
| ***E. coli* G-410-1** | 4 | 32 | 6 | 32 |
| ***E. coli* G-28-1** | 4 | 32 | 6 | 32 |
| ***E. coli* S-322-1** | 4 | 16 | 6 | 24 |
| ***E. coli* DO24*** | 0,5 | 0,5 | 2 | 2 |
| ***P. mirabilis* 5197**** | >128 | >128 | >128 | >128 |

Mit der Verwendung des CECRDM liegen die MHK-Werte der als zuvor nichtadäquat bezeichneten Methode der Colistinresistenzbestimmung (E-Test auf Platten) in der gleichen Größenordnung wie die als Referenzmethode bezeichnete Testung mittels CAB und Mikrodilution. Die Verwendung von CECRDM erlaubt es somit, auch in Laboren, die nicht für die Mikrodilutionsmethode ausgestattet sind, Colistinresistenz in Bakterien mit Plasmid-Iokalisierter Colistinresistenz und auch in Bakterien mit chromosomal lokalisierter Resistenz einfach, schnell und kostengünstig zu bestimmen.

### Abbildungslegenden

Fig. 1 zeigt die Minimale Hemm-Konzentration (MHK-Werte) von 67 *mcr-1-*kodierenden Isolaten ermittelt mittels Agardiffusion auf Cation-adjusted Mueller Hinton Medium (CAB)

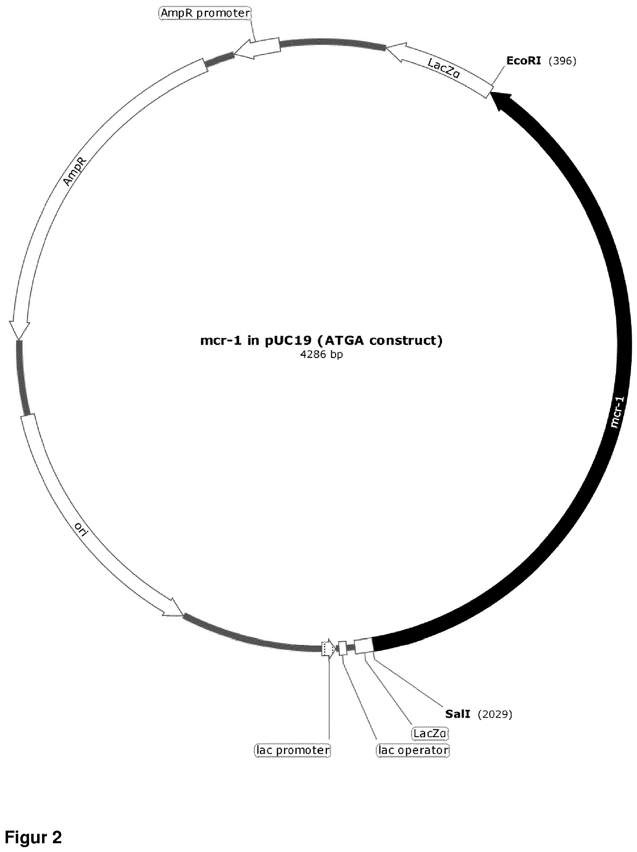

Fig. 2 zeigt die Plasmidkarte des Konstruktes *mcr-1* in pUC19.

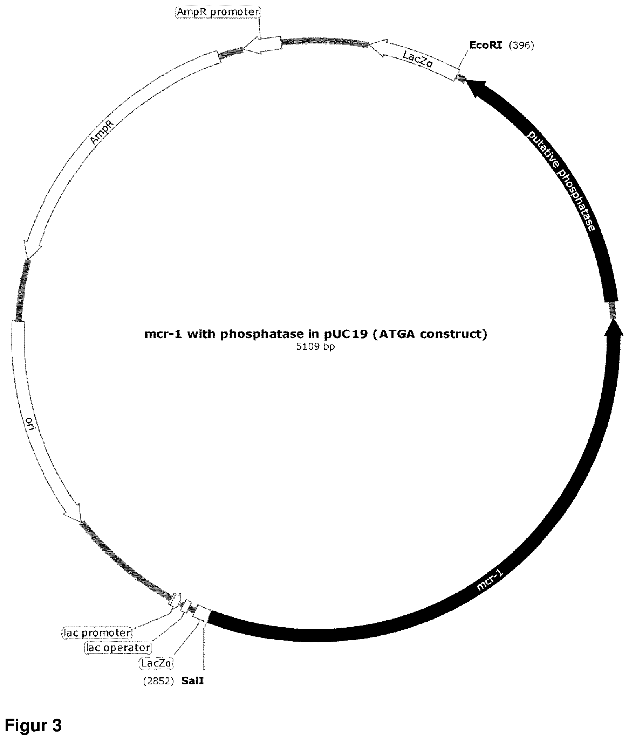

Fig. 3 zeigt die Plasmidkarte des Konstruktes *mcr-1* und Phosphatase-Gen in pUC19.

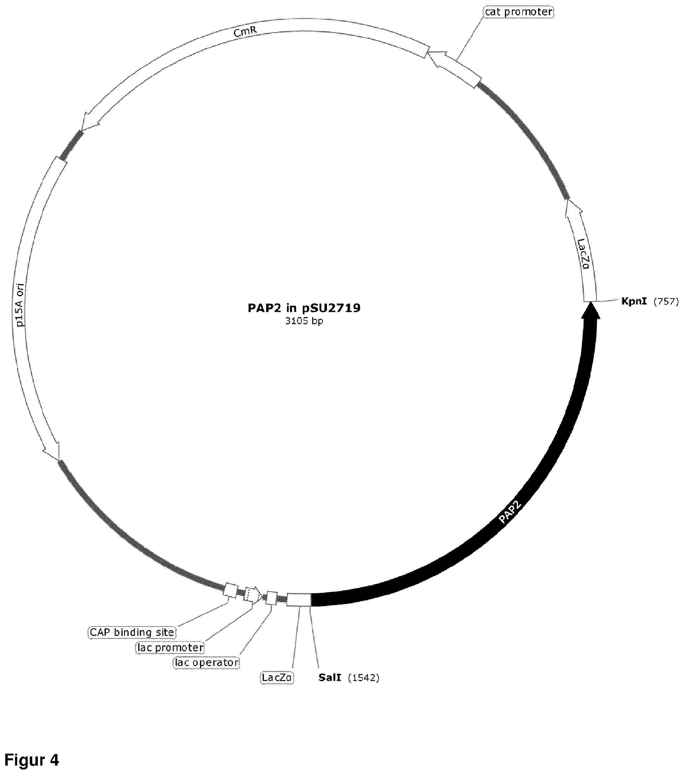

Fig. 4 zeigt die Plasmidkarte des Konstruktes Phosphatase-Gen in pSU2719.
Fig. 5 zeigt die schematische Darstellung der analysierten Konstrukte, wobei VK eine Vektorkontrolle darstellt.
Fig. 6 zeigt die beispielhafte Darstellung einer Resistenzbestimmung mittels E-Test. Sensitives Isolat ohne (A) oder mit 5 mM Calciumzugabe (B), resistentes Isolat ohne (C) oder mit 5 mM Calciumzugabe (D).
Fig. 7 zeigt die beispielhafte Darstellung der Resistenzbestimmung mittels Mikrodilution im Mikrotiterplattenformat. Mit einem weißen X markiert ist die Konzentration, die als MHK bestimmt worden ist.

## Patentansprüche

1. Verfahren zur Detektion von Colistin-resistenten Gram-negativen Bakterien in einer biologischen Probe umfassend folgende Schritte:
i) Bereitstellen eines sterilen für Gram-negative Bakterien geeigneten Nährmediums umfassend 2,5 - 20 mM Calciumionen
ii) Zugabe von Colistinsulfat in einer Konzentration von 0,125 mg/L - 512 mg/L zum Medium aus Schritt i)
iii) Zugabe einer Reinkultur eines Gram-negativen Bakteriums gewonnen aus einer biologischen Probe zum Medium nach Schritt ii)
iv) Inkubieren des Mediums mit der Reinkultur eines Gram-negativen Bakteriums nach Schritt iii)
v) Detektion von Colistin-resistenten Gram-negativen Bakterien bei Wachstum in Medium aus i) und einer Colistinsulfat-Konzentration ≥ des klinischen breakpoint.

2. Verfahren gemäß Anspruch 1 **dadurch gekennzeichnet, dass** das Nährmedium aus Schritt i) 1% Rindfleischaufguss, 1,75 % hydrolysiertes Casein und 0,15 % Stärke umfasst.

3. Verfahren gemäß einem der Ansprüche 1 bis 2 **dadurch gekennzeichnet, dass** das Nährmedium aus Schritt i) flüssig ist.

4. Verfahren gemäß einem der vorangegangenen Ansprüche **dadurch gekennzeichnet, dass** das Nährmedium aus Schritt i) zusätzlich ein Geliermittel aufweist und fest ist.

5. Verfahren gemäß einem der vorangegangenen Ansprüche **dadurch gekennzeichnet, dass** das Nährmedium aus Schritt i) Calciumionen in Form von Calcium-Chlorid aufweist.

6. Verfahren gemäß einem der vorangegangenen Ansprüche **dadurch gekennzeichnet, dass** das Nährmedium aus Schritt i) Calcium als Calciumchlorid-Dihydrat (CaCl₂ · 2 H₂O) und/oder als Calciumchlorid-Tetrahydrat (CaCl₂ · 4 H₂O) und/oder als Calciumchlorid-Hexahydrat (CaCl₂ · 6 H₂O) aufweist.

7. Verfahren gemäß einem der vorangegangenen Ansprüche **dadurch gekennzeichnet, dass** das Nährmedium aus Schritt i) Calcium in einer Konzentration von 5 mM aufweist.

8. Verfahren gemäß einem der vorangegangenen Ansprüche **dadurch gekennzeichnet, dass** die biologische Probe Material eines Menschen oder Säugetieres, wie Körperflüssigkeit, Vollblut, Serum oder Plasma, Seminalplasma, Knochenmark, Knochenmarkspunktat, Spinalflüssigkeit, peritoneale Flüssigkeit, Speichel, Tränenflüssigkeit, Gewebe, Stammzellpräparate oder Urin ist, sowie eine Stuhlprobe, Biopsiematerial, Abstriche von Körperoberflächen, eine Boden-, Wasser- oder Umweltprobe, oder eine Bewuchsprobe ist.

9. Verfahren gemäß einem der vorangegangenen Ansprüche **dadurch gekennzeichnet, dass** die Bestimmung der minimalen Hemmkonzentration (MHK-Wert) aus Schritt v) auf festem Nährmedium mittels Agardiffusionstest durchgeführt wird.

10. Verfahren gemäß einem der vorangegangenen Ansprüche **dadurch gekennzeichnet, dass** die Bestimmung der minimalen Hemmkonzentration (MHK-Wert) aus Schritt v) auf flüssigem Nährmedium mittels Dilutionsverfahren durchgeführt wird.

11. Verwendung eines Verfahrens gemäß einem der vorangegangenen Ansprüche zur Detektion von Colistin-resistenten Gram-negativen Bakterien mit Plasmidlokalisierter Colistinresistenz.

12. Verwendung eines Verfahrens gemäß einem der Ansprüche 1-10 zur Detektion von Colistin-resistenten Gram-negativen Bakterien in einer biologischen Probe.

13. Verwendung eines Verfahrens gemäß einem der Ansprüche 1-10 zur Detektion von Colistin-resistenten Gram-negativen Bakterien auf festen Nährmedien.

14. Verwendung eines Verfahrens gemäß einem der Ansprüche 1-10 zur Detektion von Colistin-resistenten Gram-negativen Bakterien in flüssigen Nährmedien.

15. Steriles für Gram-negative Bakterien geeignetes Nährmedium umfassend 2,5 - 20 mM Calciumionen und Colistinsulfat in einer Konzentration von 0,125 mg/L - 512 mg/L zur Detektion von Colistin-resistenten Gram-negativen Bakterien mit einem Verfahren gemäß einem der Ansprüche 1-10.

## Claims

1. A method for detecting colistin-resistant Gram-negative bacteria in a biological sample comprising the steps
i) Provide a sterile culture medium suitable for Gram-negative bacteria comprising 2.5 - 20 mM calcium ions
ii) Addition of colistin sulfate at a concentration of 0.125 mg/L - 512 mg/L to the medium from step i)
iii) Addition of a pure culture of a Gram-negative bacterium obtained from a biological sample to the medium of step ii)
iv) Incubating the medium with the pure culture of a Gram-negative bacterium to step iii)
v) Detection of colistin-resistant Gram-negative bacteria when growing in medium of i) and a colistin sulfate concentration ≥ of the clinical breakpoint.

2. Method according to claim 1 **characterized in that** the nutrient medium from step i) comprises 1% beef infusion, 1.75% hydrolyzed casein and 0.15% starch.

3. Method according to any of claims 1 to 2 **characterized in that** the nutrient medium from step i) is liquid.

4. Method according to one of the preceding claims **characterized in that** the nutrient medium from step i) additionally contains a gelling agent and is solid.

5. Method according to one of the preceding claims **characterized in that** the nutrient medium from step i) contains calcium ions in the form of calcium chloride.

6. Method according to one of the preceding claims **characterized in that** the nutrient medium from step i) contains calcium as calcium chloride dihydrate (CaCl2 · 2 H2O) and/or as calcium chloride tetrahydrate (CaCI2 · 4 H2O) and/or as calcium chloride hexahydrate (CaCl2 · 6 H2O).

7. Method according to one of the preceding claims **characterized in that** the nutrient medium from step i) contains calcium in a concentration of 5 mM.

8. Method according to any of the preceding claims **characterized in that** the biological sample is human or mammalian material, such as body fluid, whole blood, serum or plasma, seminal plasma, bone marrow, bone marrow puncture, spinal fluid, peritoneal fluid, saliva, tear fluid, tissue, stem cell preparations, or urine, and is a stool sample, biopsy material, body surface swab, soil, water, or environmental sample, or fouling sample.

9. Method according to one of the preceding claims **characterised in that** the determination of the minimum inhibitory concentration (MIC value) from step v) is carried out on solid nutrient medium by means of an agar diffusion test.

10. Method according to one of the preceding claims **characterized in that** the determination of the minimum inhibitory concentration (MIC value) from step v) is carried out on liquid culture medium by means of dilution method.

11. Use of a method according to any of the preceding claims for the detection of colistin-resistant Gram-negative bacteria with plasmid-localized colistin resistance.

12. Use of a method according to any one of claims 1-10 for the detection of colistin-resistant Gram-negative bacteria in a biological sample.

13. Use of a method according to any one of claims 1-10 for the detection of colistin-resistant Gram-negative bacteria on solid culture media.

14. Use of a method according to any one of claims 1-10 for the detection of colistin-resistant Gram-negative bacteria in liquid culture media.

15. Sterile nutrient medium suitable for Gram-negative bacteria comprising 2.5 - 20 mM calcium ions and colistin sulfate in a concentration of 0.125 mg/L - 512 mg/L for the detection of colistin-resistant Gram-negative bacteria by a method according to any one of claims 1-10.

## Revendications

1. Procédé de détection de bactéries Gram-négatives résistantes à la colistine dans un échantillon biologique comprenant les étapes suivantes
i) fournir un milieu de culture stérile approprié pour les bactéries Gram-négatives comprenant 2,5 - 20 mM d'ions calcium
ii) addition de sulfate de colistine à une concentration de 0,125 mg/L - 512 mg/L au milieu de l'étape (i)
iii) Ajout d'une culture pure d'une bactérie Gram-négative obtenue à partir d'un échantillon biologique au milieu de l'étape ii)
(iv) l'incubation du milieu avec la culture pure d'une bactérie Gram-négative après l'étape (iii)
v) Détection de bactéries Gram-négatives résistantes à la colistine lors de la culture dans un milieu i) et une concentration de sulfate de colistine ≥ du point d'arrêt clinique.

2. Procédé selon revendication 1, **caractérisé en ce que** le milieu nutritif de l'étape i) comprend 1 % d'infusion de bœuf, 1,75 % de caséine hydrolysée et 0,15 % d'amidon.

3. Procédé selon l'une quelconque des revendications 1 à 2 **caractérisé en ce que** le milieu nutritif de l'étape i) est liquide.

4. Procédé selon l'une des revendications précédentes, **caractérisée en ce que** le milieu nutritif de l'étape i) contient en outre un agent gélifiant et est solide.

5. Procédé selon l'une des revendications précédentes, **caractérisé en ce que** le milieu nutritif de l'étape i) comprend des ions calcium sous la forme de chlorure de calcium.

6. Procédé selon l'une des revendications précédentes, **caractérisée en ce que** le milieu nutritif de l'étape i) comprend du calcium sous forme de chlorure de calcium dihydraté · 2 H2O) et/ou de chlorure de calcium tétrahydraté · 4 H2O) et/ou de chlorure de calcium hexahydraté · 6 H2O).

7. Procédé selon l'une des revendications précédentes, **caractérisé en ce que** le milieu nutritif de l'étape i) contient du calcium à une concentration de 5 mM.

8. Procédé selon l'une des revendications précédentes, **caractérisée en ce que** l'échantillon biologique est un matériau humain ou mammifère, tel qu'un fluide corporel, du sang entier, du sérum ou du plasma, du plasma séminal, de la moelle osseuse, une ponction de moelle osseuse, du liquide céphalo-rachidien, du liquide péritonéal, de la salive, du liquide lacrymal, des tissus, des préparations de cellules souches ou de l'urine, et est un échantillon de selles, un matériau de biopsie, des écouvillons de surface corporelle, un échantillon de sol, d'eau ou d'environnement, ou un échantillon d'encrassement.

9. Procédé selon l'une des revendications précédentes, **caractérisée en ce que** la détermination de la concentration minimale inhibitrice (valeur CMI) de l'étape v) est effectuée sur un milieu nutritif solide au moyen d'un test de diffusion sur gélose.

10. Procédé selon l'une des revendications précédentes **caractérisée en ce que** la détermination de la concentration minimale d'inhibition (valeur CMI) de l'étape v) est effectuée sur un milieu nutritif liquide au moyen de méthodes de dilution.

11. Utiliser une méthode conforme à l'une des revendications précédentes pour la détection des bactéries Gram-négatives résistantes à la colistine avec une résistance à la colistine localisée dans le plasmide.

12. Utiliser une méthode selon l'une des revendications 1 à 10 pour détecter les bactéries Gram-négatives résistantes à la colistine dans un échantillon biologique.

13. Utilisation d'une méthode selon l'une des revendications 1 à 10 pour la détection de bactéries Gram-négatives résistantes à la colistine sur des milieux de culture solides.

14. Utilisation d'une méthode selon l'une des revendications 1 à 10 pour la détection des bactéries Gram-négatives résistantes à la colistine dans les nutriments liquides.

15. Milieu nutritif stérile approprié pour les bactéries Gram-négatives comprenant 2,5 - 20 mM d'ions calcium et du sulfate de colistine à une concentration de 0,125 mg/L - 512 mg/L pour la détection des bactéries Gram-négatives résistantes à la colistine par une méthode selon l'une des revendications 1 à 10.
